# EUROPÄISCHE PATENTSCHRIFT

(11) **EP 1 430 907 B1**
(45) Veröffentlichungstag und Bekanntmachung des Hinweises auf die Patenterteilung: **05.11.2008**
(21) Anmeldenummer: 03090438.7
(22) Anmeldetag: 15.12.2003
(51) Int. Cl.: A61K 8/97, A61Q 19/00

(54) **Relax-Kosmetikum mit Temperatureffekt**
Relaxing cosmetic with temperature effect
Composition cosmétique relaxante avec effet de température

(30) Priorität: 19.12.2002 DE 10261815
(43) Veröffentlichungstag der Anmeldung: 23.06.2004
(73) Patentinhaber: Coty B.V., 2031 CC Haarlem (NL)
(72) Erfinder: Golz-Berner, Karin, 98000 Monaco (MC); Zastrow, Leonhard, 98000 Monaco (MC)
(74) Vertreter: Walter, Wolf-Jürgen

(56) Entgegenhaltungen:
- EP-A- 1 215 257
- IT-B- 1 243 593
- DATABASE WPI Section Ch, Week 199514 Derwent Publications Ltd., London, GB; Class B04, AN 1995-101794 XP002274876 & JP 07 025763 A (SANSEI SEIYAKU KK) 27. Januar 1995 (1995-01-27)
- MARTINI M-C ET AL: "MENTHE" 1992, ACTIFS ET ADDITIFS EN COSMETOLOGIE, PARIS, LAVOISIER TEC & DOC, FR, PAGE 21 , XP002061713 * das ganze Dokument *
- D'AMELIO F: "Botanicals - A Phytocosmetic Desk Reference" 1999, BOTANICALS. A PHYTOCOSMETIC DESK REFERENCE, XX, XX, PAGES 168, 169, 180 , XP002274875 * das ganze Dokument *

## Beschreibung

Die Erfindung betrifft neue kosmetische Relax-Zubereitungen mit Temperatur-Wechselwirkungen auf der Haut.

Es ist seit langem bekannt, Auszüge von Pfefferminze (Menthae) in Süßwaren, Zigaretten, Eiskrem und Liköre einzubringen, aber auch in kosmetische Zubereitungen, z.B. Mundhygienemittel, Toilettenartikel, Parfüms. Das Öl wird insbesondere bei Erkrankungen der oberen Luftwege, Hautreizungen, neuralgischen Schmerzen und Verdauungsbeschwerden verwendet.

Kosmetisch interessant sind besonders die kühlenden und erfrischenden Wirkungen der Minze sowie antibakterielle Wirkungen.

Mohn (Papaveraceae) ist medizinisch wegen seines Alkaloidgehaltes bekannt geworden (Morphium, Kodein); darüber hinaus in der ayurvedischen Medizin die Samen bei Verdauungsstörungen. Die WO 00/76458 beschreibt ein enzymhaltiges kosmetisches Produkt, das u.a. das pflanzliche Milchwasser von Mohn zusammen mit Kokosmilch und Reisschalen- bzw. Reiskeimöl enthält.

Das IT-Patent 01243593 betrifft ein kosmetisches Tonikum, das u.a. wässrige Extrakte von Papaver und Minze enthält, wobei der Papaver-Gehalt bei 30 % liegt.

Die JP-07025763 offenbart ein Hautaufhellungsmittel für Sommersprossen und Altersflecken, enthalten Kojisäure und einen Pflanzenextrakt bzw. Extraktgemisch. Es sind u.a. auch Minze und Papaver genannt. Die EP 1 215 257 betrifft ein wärmendes Mittel, das neben anderen Bestandteilen auch Minze enthalten kann. Martini M-C et al. "Menthe" 1992, Actifs et Additifs en Cosmétologie, Paris, Lavosier Tec & Doc, FR, S. 21 beschreiben Minze als kühlendes Mittel in Kosmetika. Die Verwendung von Minzeextrakten und auch von Papaverextrakten wird allgemein in D'Amelio F. "Botanicals - A phytocosmetic desk reference" 1999, Botanicals, S. 168, 169, 180 beschrieben.

Der Erfindung liegt die Aufgabe zugrunde, ein neues kosmetisches Mittel mit relaxierender und temperierender Wirkung bereitzustellen.

Erfindungsgemäß enthält das Kosmetikum 0,1 bis 10 Gew-% eines Extraktes der Blütenblätter, Samenkapseln oder eines Gemisches davon von Papaveraceae und 0,1 bis 10 Gew-% eines Extraktes der oberirdischen Teile von Menthae, zusammen mit 80-99,8 Gew-% kosmetischen Hilfs-, Träger-, Wirkstoffen oder Gemischen davon, jeweils bezogen auf das Gesamtgewicht des Kosmetikums.

Bei den Extrakten von Papaveraceae sind bevorzugt die Extrakte der Blütenblätter. Bevorzugte Mohnarten sind Papaver rhoeas und Papaver somniferum, wobei Papaver rhoeas besonders bevorzugt ist.

Bei den Extrakten von Menthae können die Blätter und Stengel eingesetzt werden. Bevorzugt sind Extrakte der Blätter. Bevorzugte Minze-Arten sind Mentha aquatica, Mentha arvensis und Mentha piperita.

Als Extraktionsmittel für die erfindungsgemäßen Zwecke können Wasser, niedere Alkohole wie Ethanol oder mehrwertige Alkohole wie Propylenglycol oder Gemische von Propylenglycol mit Glycerin eingesetzt werden. Für Parfümzubereitungen sind alkoholische Extrakte bevorzugt.

Eine besondere Ausführungsform der Erfindung besteht darin, daß der Extrakt von Papaveraceae und der Extrakt von Menthae ein alkoholischer Extrakt ist, und das Kosmetikum in Form eines Parfüms vorliegt mit Konzentrationen der Wirkstoffe jeweils im Bereich von 2,5 bis 10 Gew-%.

Eine weitere bevorzugte Ausführungsform der Erfindung ist ein Relax-Kosmetikum, bei dem der Extrakt von Papaveraceae und der Extrakt von Menthae zusammen mit einem selbstemulgierenden Silicongel vorliegt, wobei die Extrakte in dem selbstemulgierenden Silicongel zusätzlich in verkapselter Form vorliegen können. Sie können jedoch auch in die Gelstruktur unverkapselt eingearbeitet sein.

Es wurde gefunden, daß die erfindungsgemäße kosmetische Zubereitung einen deutlichen Kühleffekt auf der Haut erzeugt, der kurze Zeit danach (einige Sekunden je nach Hauttyp) von einem ebenso deutlichen Wärmeeffekt abgelöst wird. Nach der anfänglichen Kühlwirkung führt der danach auftretende und dann langsam abklingende Wärmeeffekt zu einer deutlichen entspannenden relaxierenden Hautwirkung, die beim Anwender eine insgesamt entspannende Wirkung erzielt. In der speziellen Kombination mit einem selbstemulgierenden Silicongel wird die Gesamtaktivität der erfindungsgemäß eingesetzten Wirkstoffe Minze und Mohn nochmals zusätzlich erhöht, und es tritt eine Hautstraffung auf. Insbesondere mit dem Silicongel tritt ein Entspannungseffekt für die Haut auf, den die Einzelkomponenten in dieser Form nicht zeigen.

Besonders merkliche Hauteffekte werden dann erzielt, wenn die Konzentrationen in dem Kosmetikum, das kein Parfüm ist, von Minze im Bereich von 1,5 bis 6 Gew-% liegen und die von Mohn im Bereich von 1 bis 4 Gew-%, jeweils bezogen auf die Trockensubstanz der Extrakte.

Das erfindungsgemäße Kosmetikum enthält weiterhin kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdikungsmittel, Duftstoffe, Alkohole, Polyole, Ester, Elektrolyte, Gelbildner, polare und unpolare Öle, Polymere, Copolymere, Emulgatoren, Wachse, Stabilisatoren, Erweichungsmittel.

Die für die Erfindung eingesetzten Öle können übliche kosmetische Öle sein, wie ein Mineralöl; Siliconöle; hydriertes Polyisobuten; synthetisches oder aus Naturprodukten hergestelltes Squalan ); kosmetische Ester oder Ether, die verzweigt oder unverzweigt, gesättigt oder ungesättigt sein können; pflanzliche Öle; oder Gemische zweier oder mehrerer davon.

Besonders geeignete Öle sind Siliconöle. Für die Herstellung von Wasser/Siliconöl-Emulsionen (W/S) werden bevorzugt selbstemulgierende Silicongele verwendet, wie KSG-21® oder KSG-210® (von Shin-Etsu Silicones of America, Akron, Ohio, USA)

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Es können auch weitere Wirkstoffe enthalten sein, wie z.B. anorganische und organische Lichtschutzmittel, Radikalfänger, Feuchthaltemittel, Vitamine, Enzyme, pflanzliche Wirkstoffe, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Ein weiterer Zusatz für das erfindungsgemäße Kosmetikum ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser gemäß WO99/66881.

Zu den genannten Antioxidationsmitteln und Radikalfängern gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure.

Besonders vorteilhaft ist ein Gemisch aus Enzymen und Vitaminen, das ein durch Ultraschallbehandlung hergestelltes Aufschlußprodukt einer Hefe darstellt, wobei das Aufschlußprodukt SOD, Protease, Vitamin B₂, Vitamin B₆, Vitamin B₁₂, Vitamin D₂ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Polyole sind ebenfalls mögliche Bestandteile des erfindungsgemäßen Kosmetikums. Polyole sind z.B Propylenglycol, Dipropylenglycol, Ethylenglycol, Isoprenglycol, Glycerin, Butylenglycole, Sorbitol und Gemische davon. Der Anteil des Polyols liegt im Bereich von 0,1 bis 40 Gew-%, vorzugsweise von etwa 5% bis etwa 20 Gew-% der Zusammensetzung.

Als Feuchthaltemittel können verwendet werden Glycerin, Butylenglycol, Propylenglycol und Gemische davon.

Die kosmetischen Wirkstoffe, insbesondere Minze und Mohn, können in übliche Liposome als Transportsystem verkapselt werden. Liposome sind vollständig geschlossene Lipid-Bilayer-Membranen, die ein wäßriges Volumen eingeschlossen enthalten. Liposome können unilamellare Vesikel sein (die eine Einzelmembran-Bilayer besitzen) oder multilamellare Vesikel (Onion-ähnliche Strukturen, gekennzeichnet durch Mehrfachmembran-Bilayer, von denen jede von der nächsten durch eine wäßrige Schicht getrennt ist). Die Herstellung von Liposomen, aus gesättigten und ungesättigten Lipiden, ist in sehr vielen Patenten beschrieben worden, ebenso deren Einsatz als Transportsystem. Die Einarbeitung darin kann auf übliche Weise erfolgen.

Derartige Liposome werden meist aus Phospholipiden hergestellt. Die Verkapselung der erfindungsgemäßen Wirkstoffe kann jedoch auch in Alginaten oder anderen gelartigen Strukturen erfolgen.

Die Verwendung der erfindungsgemäßen kosmetischen Zusammensetzungen kann z.B. erfolgen in Form von Gelen, After-sun-Produkten, Tagescremes, Nachtcremes, Masken, Körperlotionen, Reinigungsmilch, Duschgelen, Duschölen, Badeölen; Eau de Toilette, Eau de Parfüm, Parfüms, Sonnenprodukte für den Winter und in Produkten der dekorativen Kosmetik wie Deo-Stiften, Parfüm-Stiften, Lippenstiften, Kompaktprodukten wie Rouge, Grundierung, Make-up usw. Die Herstellung derartiger Produkte erfolgt auf eine Weise, wie sie dem Fachmann auf diesem Gebiet bekannt ist.
Ein weiterer Gegenstand der Erfindung ist ein Verfahren zur Herstellung eines Relax-Kosmetikums, indem ein Extrakt von Papaveraceae und von Menthae, der in eine Pulverform mit Teilchengrößen von 0,1 bis 25 µm überführt wird, in wenig Wasser gelöst und mit einem selbstemulgierenden Silicongel bei einer Temperatur von 60 bis 65°C und für einen Zeitraum von 5 bis 20 Minuten mit einer Geschwindigkeit von 400 bis 1000 U/Min, vorzugsweise 600-1000 U/min vermischt wird. Dadurch erhält man ein Gel, bei dem die Wirkstoffe in ihrer wirksamen Struktur erhalten bleiben und das auch bei geringeren Konzentrationen von 0,1 bis 1,5 Gew-% noch merkliche Hautwirksamkeiten zeigt.

Die Erfindung soll nachstehend durch Beispiele näher erläutert werden. Alle Angaben erfolgen in Gewichtsprozent, sofern nichts anderes angegeben ist.

### Beispiel 1 Gelcreme Silicon-Wasser

| **Phase A** | |
|---|---|
| Wasser | q.s. ad 100 |
| Glycerine | 1,0 |
| Disodium Hydrogenphosphate | 0,5 |
| Sodium Chloride | 1,5 |

| **Phase B** | |
|---|---|
| Silicone | 2,0 |
| Silicongel KSG-210® | 3,0 |

| **Phase C** | |
|---|---|
| Ethanol | 10,0 |

| **Phase D** | |
|---|---|
| Mentha piperita-Extrakt (wäßrig) | 3,0 |
| Papaver rhoeas-Extrakt (wäßrig) | 3,0 |

| **Phase E** | |
|---|---|
| Parfüm | 0,2 |
| Konservierungsmittel | 0,5 |

Nach separater Herstellung der Phase A bei maximal 65°C und der Phase B bei maximal 60°C werden beide miteinander unter Rühren vermischt und weitere 10 Minuten bei 800-1000°C gerührt. Nach dem Abkühlen und sehr langsamem Rühren wird bei 30°C die Phase C hinzugegeben und unter Rühren auf 25°C abgekühlt.

Die Phase D wird mit etwa 10 % der Gesamtmenge Wasser und Zugabe der trockenen Extraktpulver unter Rühren mit 50-100 U/min hergestellt und dann zu dem Gemisch ebenfalls unter Rühren zugegeben. Schließlich wird die Phase E hinzugesetzt, und das Gesamtgemisch 10 Minuten bei etwa 1000 U/min gerührt.

### Beispiel 2 Parfüm

| | |
|---|---|
| Wasser | 1,5 |
| Parfümöl | 13,0 |
| Ethanol | q.s. ad 100 |
| Mentha arvensis-Extrakt (alkohol.) | 3,0 |
| Papaver rhoeas-Extrakt (alkohol.) | 3,0 |

### Beispiel 3 Sonnenprodukt für Wintersonne SPF 15

| **Phase A** | |
|---|---|
| Wasser | q.s. ad 100 |
| Glycerine | 1,0 |
| Disodium Hydrogenphosphate | 0,5 |
| Sodium Chloride | 1,5 |

| **Phase B** | |
|---|---|
| Cyclohexasiloxane | 5,0 |
| Uvinul MC 80 | 5,0 |
| Parsol 1768 | 3,0 |
| Escalol 587 | 5,0 |
| Silicongel KSG-210® | 3,0 |

| **Phase C** | |
|---|---|
| Ethanol | 2,0 |
| Mentha piperita- und Papaver rhoeas-Extrakt in Liposomen (1:1) | 5,0 |
| Parfümöl | 0,5 |
| Konservierungsmittel | 0,5 |
| Wirkstoffkomplex mit Radikalschutzfaktor gemäß Beispiel 1 WO99/66881 | 10,0 |

Nach Vermischen der getrennt hergestellten Phasen A und B wird unter Rühren auf 30°C abgekühlt und die Phase C hinzugegeben. Das Gemisch wird bei etwa 1000 U/Min für 20 Minuten gerührt.

### Beispiel 4 After sun-Lotion

| **Phase A** | |
|---|---|
| Wasser | q.s. ad 100 |
| Glycerine | 1,0 |
| Disodium Hydrogenphosphate | 0,5 |
| Sodium Chloride | 0,8 |

| **Phase B** | |
|---|---|
| Silicone | 1,0 |
| Silicongel KSG-210® | 2,0 |

| **Phase C** | |
|---|---|
| Mentha aquatica-Extrakt (alkohol.) | 2,0 |
| Papaver somniferum-Extrakt (alkohol.) | 2,0 |
| Parfümöl | 0,5 |
| Konservierungsmittel | 0,5 |

Es wird wie im Beispiel 1 gearbeitet.

## Patentansprüche

1. Relax-Kosmetikum mit Temperatureffekt, **gekennzeichnet durch** einen Gehalt an
0,1 bis 10 Gew-% eines Extraktes der Blütenblätter, Samenkapseln oder eines Gemisches davon von Papaveraceae und 0,1 bis 10 Gew-% eines Extraktes der oberirdischen Teile von Menthae,
zusammen mit 80-99,8 Gew-% kosmetischen Hilfs-, Träger-, Wirkstoffen oder Gemischen davon, jeweils bezogen auf das Gesamtgewicht des Kosmetikums.

2. Relax-Kosmetikum nach Anspruch 1, **dadurch gekennzeichnet, daß** der Extrakt von Papaveraceae ein wäßriger Extrakt der Blütenblätter ist.

3. Relax-Kosmetikum nach Anspruch 1, **dadurch gekennzeichnet, daß** der Extrakt von Papaveraceae und der Extrakt von Menthae zusammen mit einem selbstemulgierenden Silicongel vorliegt.

4. Relax-Kosmetikum nach Anspruch 3, **dadurch gekennzeichnet, daß** die Extrakte in dem selbstemulgierenden Silicongel in verkapselter Form vorliegen.

5. Relax-Kosmetikum nach Anspruch 1, **dadurch gekennzeichnet, daß** der Extrakt von Papaveraceae und der Extrakt von Menthae ein alkoholischer Extrakt ist, und das Kosmetikum in Form eines Parfüms vorliegt mit Konzentrationen der Wirkstoffe jeweils im Bereich von 2,5 bis 10 Gew-%.

6. Verfahren zur Herstellung eines Relax-Kosmetikums nach Anspruch 1, **dadurch gekennzeichnet, daß** ein Extrakt von Papaveraceae und von Menthae, der in eine Pulverform mit Teilchengrößen von 0,1 bis 25 µm überführt wird, in wenig Wasser gelöst und mit einem selbstemulgierenden Silicongel bei einer Temperatur von 60 bis 65°C und für einen Zeitraum von 5 bis 20 Minuten mit einer Geschwindigkeit von 600 bis 1000 U/Min vermischt wird.

## Claims

1. A relax cosmetic having a temperature effect which comprises
0.1-10 % by weight of an extract from the petals, seed capsules or a mixture thereof of Papaveraceae and 0.1-10 % by weight of an extract from the overground parts of Menthae, along with 80-99,8 % by weight of cosmetic auxiliaries, carrier substances, active agents or mixtures thereof, all percentages being relative to the cosmetic's total weight.

2. A relax cosmetic according to Claim 1, wherein the Papaveraceae extract is an aqueous extract obtained from the petals.

3. A relax cosmetic according to Claim 1, wherein the Papaveraceae and Menthae extracts are provided mixed with a self-emulsifying silicone gel.

4. A relax cosmetic according to Claim 3, wherein said extracts contained in the self-emulsifying silicone gel are provided in an encapsulated form.

5. A relax cosmetic according to Claim 1, wherein the Papaveraceae and Menthae extracts are alcoholic extracts and the cosmetic is provided in the form of a perfume in which the active agents are contained in amounts ranging between 2.5 and 10 % by weight each.

6. A method for producing a relax cosmetic according to claim 1, comprising the steps of making extracts of Papaveraceae and Menthae into a powder whose particle size ranges between 0.1 and 25 µm, dissolving said powder in a little water and mixing it with a self-emulsifying silicone gel at a temperature of 60-65° C for 5-20 minutes and at a rate of 600-1,000 rpm.

## Revendications

1. Composition cosmétique relaxante à effet de température,
**caractérisée par** une teneur de 0,1 à 10 % en poids d'un extrait de pétales, de capsules de graines ou leur mélange de Papaveraceae et de 0,1 à 10 % en poids d'un extrait des parties aériennes de Menthae, conjointement avec 80 à 99,8 % en poids d'adjuvants, de véhicules, de principes actifs cosmétiques ou leurs mélanges, respectivement par rapport au poids total de la composition cosmétique.

2. Composition cosmétique relaxante selon la revendication 1,
**caractérisée en ce que** l'extrait de Papaveraceae est un extrait aqueux de pétale.

3. Composition cosmétique relaxante selon la revendication 1,
**caractérisée en ce que** l'extrait de Papaveraceae et l'extrait de Menthae se présente conjointement avec un gel auto-émulsifiant de silicone.

4. Composition cosmétique relaxante selon la revendication 3,
**caractérisée en ce que** les extraits se présentent sous forme encapsulée dans le gel de silicone auto-émulsifiant.

5. Composition cosmétique relaxante selon la revendication 1,
**caractérisé en ce que** l'extrait de Papaveraceae et l'extrait de Menthae sont un extrait alcoolique et la composition cosmétique se présente sous forme d'un parfum présentant une concentration de principes actifs respectivement de l'ordre de 2,5 à 10 % en poids.

6. Procédé de production d'une composition cosmétique relaxante selon la revendication 1, **caractérisé en ce qu'**un extrait de Papaveraceae et de Menthae qui est converti en une forme pulvérulente présentant une taille de particules de 0,1 à 25 µm est dissous dans un peu d'eau et est mélangé avec un gel de silicone auto-émulsifiant à une température de 60 à 65° C et pendant une durée de 5 à 20 minutes à une vitesse de 600 à 1000 U/min.
